# EUROPEAN PATENT SPECIFICATION

(11) **EP 2 056 867 B1**
(45) Date of publication and mention of the grant of the patent: **27.03.2013**
(21) Application number: 07786549.1
(22) Date of filing: 03.08.2007
(51) Int. Cl.: A61K 38/57, A61P 37/00

(54) **CYSTATIN FROM PARASITIC NEMATODES IN THE TREATMENT OF AUTOIMMUNE OR ALLERGIC DISEASES**
CYSTATIN AUS PARASITÄREN NEMATODEN BEI DER BEHANDLUNG VON AUTOIMMUNKRANKHEITEN ODER ALLERGISCHEN ERKRANKUNGEN
CYSTATINE DE NÉMATODES PARASITES POUR LE TRAITEMENT DE MALADIES AUTOIMMUNES OU ALLERGIQUES

(30) Priority: 04.08.2006 EP 06016356; 04.04.2007 US 910118 P
(43) Date of publication of application: 13.05.2009
(73) Proprietor: Hartmann, Susanne, 12307 Berlin (DE); Schnoeller, Corinna, 10407 Berlin (DE); Lucius, Richard, 10717 Berlin (DE); Hamelmann, Eckard, 12203 Berlin (DE)
(72) Inventor: HARTMANN, Susanne, 12307 Berlin (DE); LUCIUS, Richard, 10717 Berlin (DE); SCHNOELLER, Corinna, 10407 Berlin (DE); HAMELMANN, Eckard, 12203 Berlin (DE)
(74) Representative: Engelhard, Markus
(86) International application number: PCT/EP2007/006888
(87) International publication number: WO 2008/015014

(56) References cited:
- EP-A2- 0 373 771
- WO-A2-02/36733
- WO-A2-02/085930
- US-A1- 2002 086 276
- SCHÖNEMEYER A ET AL: "Modulation of human T cell responses and macrophage functions by onchocystatin, a secreted protein of the filarial nematode Onchocerca volvulus." JOURNAL OF IMMUNOLOGY (BALTIMORE, MD. : 1950) 15 SEP 2001, vol. 167, no. 6, 15 September 2001 (2001-09-15), pages 3207-3215, XP002413959 ISSN: 0022-1767
- HARTMANN S ET AL: "Modulation of host immune responses by nematode cystatins." INTERNATIONAL JOURNAL FOR PARASITOLOGY, vol. 33, no. 11, 2003, pages 1291-1302, XP002413960 ISSN: 0020-7519
- SCHIERACK PETER ET AL: "Parasite-specific immunomodulatory functions of filarial cystatin." INFECTION AND IMMUNITY, vol. 71, no. 5, May 2003 (2003-05), pages 2422-2429, XP002413961 ISSN: 0019-9567
- HARTMANN SUSANNE ET AL: "A filarial cysteine protease inhibitor down-regulates T cell proliferation and enhances interleukin-10 production" EUROPEAN JOURNAL OF IMMUNOLOGY, vol. 27, no. 9, 1997, pages 2253-2260, XP009076811 ISSN: 0014-2980
- MANOURY BENEDICTE ET AL: "Bm-CPI-2, a cystatin homolog secreted by the filarial parasite Brugia malayi, inhibits class II MHC-restricted antigen processing" CURRENT BIOLOGY, vol. 11, no. 6, 20 March 2001 (2001-03-20), pages 447-451, XP002413963 ISSN: 0960-9822

### SEQUENCE LISTING

<110> Charité Universitatsmedizin Berlin, Humboldt-Universität zu Berlin
<120> Uses of cystatin
<130> U30193PCT
<160> 3
<170> PatentIn version 3.3
<210> 1
   <211> 157
   <212> PRT
   <213> Acanthocheilonema viteae
<400> 1
   Met Met Leu Ser Ile Lys Glu Asp Gly Leu Leu Val Val Leu Leu Leu
   1               5                   10                  15
   Ser Phe Gly Val Thr Thr Val Leu Val Arg Cys Glu Glu Pro Ala Asn
               20                  25                  30
   Met Glu Ser Glu Val Gln Ala Pro Asn Leu Leu Gly Gly Trp Gln Glu
           35                  40                  45
   Arg Asn Pro Glu Glu Lys Glu Ile Gln Asp Leu Leu Pro Lys Val Leu
       50                  55                  60
   Ile Lys Leu Asn Gln Leu Ser Asn Val Glu Tyr His Leu Met Pro Ile
   65                  70                  75                  80
   Lys Leu Leu Lys Val Ser Ser Gln Val Val Ala Gly Leu Arg Tyr Lys
                   85                  90                  95
   Met Glu Ile Gln Val Ala Gln Ser Glu Cys Lys Lys Ser Ser Gly Glu
               100                 105                 110
   Glu Val Asn Leu Lys Thr Cys Lys Arg Leu Glu Gly His Pro Asp Gln
           115                 120                 125
   Ile Ile Thr Leu Glu Ala Trp Glu Lys Ser Trp Glu Asn Phe Leu Gln
       130                 135                 140
   Val Lys Ile Leu Glu Lys Lys Glu Val Leu Ser Ser Val
   145                 150                 155
<210> 2
   <211> 162
   <212> PRT
   <213> Onchocerca volvulus
<400> 2
   Met Leu Thr Ile Lys Asp Gly Thr Leu Leu Ile His Leu Leu Leu Phe
   1               5                   10                  15
   Ser Val Val Ala Leu Val Gln Leu Gln Gly Ala Lys Ser Ala Arg Ala
               20                  25                  30
   Lys Asn Pro Ser Lys Met Glu Ser Lys Thr Gly Glu Asn Gln Asp Arg
           35                  40                  45
   Pro Val Leu Leu Gly Gly Trp Glu Asp Arg Asp Pro Lys Asp Glu Glu
       50                  55                  60
   Ile Leu Glu Leu Leu Pro Ser Ile Leu Met Lys Val Asn Glu Gln Ser
   65                  70                  75                  80
   Asn Asp Glu Tyr His Leu Met Pro Ile Lys Leu Leu Lys Val Ser Ser
                   85                  90                  95
   Gln Val Val Ala Gly Val Lys Tyr Lys Met Asp Val Gln Val Ala Arg
               100                 105                 110
   Ser Gln Cys Lys Lys Ser Ser Asn Glu Lys Val Asp Leu Thr Lys Cys
           115                 120                 125
   Lys Lys Leu Glu Gly His Pro Glu Lys Val Met Thr Leu Glu Val Trp
       130                 135                 140
   Glu Lys Pro Trp Glu Asn Phe Met Arg Val Glu Ile Leu Gly Thr Lys
   145                 150                 155                 160
   Glu Val
<210> 3
   <211> 161
   <212> PRT
   <213> Brugia malayi
<400> 3
   Met Met Ser Thr Met Ser Ile Lys Glu Gly Leu Leu Val Ile Leu Leu
   1               5                   10                  15
   Ser Leu Phe Leu Phe Asp Thr Thr Ala Leu Ile His Arg Arg Glu Ile
               20                  25                  30
   Pro His Met Glu Ser Lys Gly Gln Met Gln Arg Gly Gln Val Leu Leu
           35                  40                  45
   Gly Gly Trp Gln Glu Arg Ser Pro Glu Asp Asn Glu Ile Leu Glu Leu
       50                  55                  60
   Leu Pro Ser Val Leu Thr Lys Val Asn Gln Gln Ser Asn Asp Glu Tyr
   65                  70                  75                  80
   His Leu Met Pro Ile Lys Leu Leu Lys Val Ser Ser Gln Val Val Ala
                   85                  90                  95
   Gly Val Lys Tyr Lys Met Glu Val Gln Val Ala Arg Ser Glu Cys Lys
               100                 105                 110
   Lys Ser Ala Ser Glu Gln Val Asn Leu Lys Thr Cys Lys Lys Leu Glu
           115                 120                 125
   Gly His Pro Asp Gln Val Met Thr Leu Glu Val Trp Glu Lys Pro Trp
       130                 135                 140
   Glu Asp Phe Leu Gln Val Asn Ile Leu Glu Thr Lys Val Leu Ser Ser
   145                 150                 155                 160
   Val

## Description

The present invention relates to uses of cystatins derived from parasitic nematodes and to a method of screening using such cystatin.

Diseases which are characterized by the presence of undesirable inflammation and inflammatory symptoms contribute significant losses of life expectancy and well-being to a large number of humans. Moreover, they represent a significant factor in damage to national economies in that virtually all human beings at some stage in their life are subject to undesirable inflammations. Treatment of inflammatory diseases in the past has been more or less non-specific, based on broad-spectrum immuno-suppressant drugs, such as corticosteroids. Although beneficial, these drugs have significant side effects which limit their use.

More recently, research has identified a number of ways to specifically block known targets in inflammation. The most successful of these approaches so far has been to block TNF alpha, a hormone-like molecule which signals information from one cell to other cells in the body. Such molecules are collectively known as cytokines. Cytokines like TNF alpha play an essential role in normal immunity, but in disease situations their levels are elevated or reduced and they exert deleterious effects on cell function and hence the well-being of the individual. In certain disease situations, such as colitis and rheumatoid arthritis, blockage of TNF alpha has been associated with some benefit in patients. Despite these successes, currently available TNF alpha treatments are ineffective in approximately half of the patients and in most cases are administered with other drugs. They must be given by injection and are often associated with adverse injection side reactions as well as inconvenience, and can be very costly, potentially limiting their availability to many patients.

One subset of inflammatory diseases are allergic diseases. Allergic diseases are characterized by adverse reactions to normally harmless substances, such as dust, pollen, food or mould. The immune systems of people with allergic diseases overreact to these substances. People who are sensitive to such triggering substances have a high amount of IgE in their blood. If small amounts of the triggering substances, such as pollen granules, meet with IgE in the body of a patient, the body overreacts. The immune system tries to fight the substance that is thus recognized as non-self. This results in allergic symptoms, such as swelling, tearing, congestion, sneezing and other symptoms. Examples of allergic conditions are allergic rhinitis, also referred to as "hay fever", asthma, atopic dermatitis, allergic sinusitis, food allergies. Treatment of allergies has focussed on environmental control, pharmacological therapy and allergen immunotherapy. Environmental control involves avoiding exposure to the triggering substances, however, can only be successful to a certain extent. Pharmacological therapy is mostly a symptomatic treatment. Allergen immunotherapy is a way of desensitizing a patient's immune system by improving the way the immune system responds to an allergic trigger. None of these approaches have been overtly successful. Accordingly, there exists a need in the art to improve current therapies for allergic diseases.

Autoimmune diseases are another subgroup of inflammatory diseases and are accompanied by inflammatory symptoms. Autoimmune diseases are characterized by a misguided immune system, wherein the patient's body or parts thereof is attacked by the immune system and thereby damaged. Autoimmune diseases are characterized by the body's immune response being directed against its own tissues causing prolonged inflammation and subsequent tissue destruction. Autoimmune diseases can cause immune-response of cells to attack the linings of joints or specific types of cells within specific tissues, thereby leading to diseases such as rheumatoid arthritis or insulin-dependent diabetes. Autoimmune diseases include, but are not limited to celiac disease, Crohn's disease, pancreatitis, lupus erhythematosus, psoriasis, multiple sclerosis, inflammatory bowel diseases, Sjogren's syndrome, Hashimoto's thyroiditis etc.

Allergic diseases and autoimmune diseases are characterized by a common etiology, in that in both cases, the immune system is misguided and aims at foreign agents as allegedly harmful agents (which they are not) or aims at the organism's own tissues as allegedly foreign agents (which they are not). In both instances, the effect of such misguidance is a long-term damage to the organism's body by inflammatory cells like neutrophils, eosinophils or macrophages that are attracted to the site of injury and activated, which leads to production of toxic molecules and the damage and destruction of the body's own cells. Consequently, therapeutical approaches to both groups of diseases have common elements, and may target at the differentiation, attraction and activation of inflammatory cells.

None of the current therapies for allergic diseases and/or autoimmune diseases are particularly successful. Accordingly, there exists a need in the art to provide for new ways of therapy for allergic and autoimmune diseases. In particular one object of the present invention was to provide for new ways of therapy of allergic diseases of the respiratory organs, such as asthma, hay fever, allergic sinusitis, allergic rhinitis. Furthermore, it was an object of the present invention to provide for ways of therapy for allergic diseases of the gastrointestinal system, such as food allergies, such as celiac disease, lactose intolerance. Moreover, it was an object of the present invention to provide for ways of treating allergic disease of the skin, such as atopic dermatitis, and/or systemic allergic diseases, such as anaphylactic reactions. Moreover, it was an object of the present invention to provide for new ways of treating autoimmune disease of the joints and/or skin and/or internal organs, such as rheumatoid arthritis, psoriasis, lupus erythematosus, multiple sclerosis and inflammatory bowel disease, such as colitis, e.g. colitis ulcerosa, and Crohn's disease.

All these objects are solved by the use of a cystatin derived from a parasitic nematode for the manufacture of a medicament for the prevention and/or treatment of an allergic and/ or autoimmune disease in a patient.

Said cystatin is derived from a parasitic nematode.

Preferably, said parasitic nematodes is parasitic to humans. In another embodiment said parasitic nematode is parasitic to animals. The term "animal", as used herein, refers to non-human animals. Preferably said parasitic nematode is parasitic to canine animals, preferably dogs, or is parasitic to rodents, preferably mice, or is parasitic to feline animals, preferably cats.

Preferably, said nematode is selected from the group comprising Onchocerca volvulus, Brugia malayi, Wuchereria bancrofti, Loa loa, Acanthocheilonema viteae, Dirofilaria immitis, Dirofilaria repens, Nippostrongylus brasiliensis and Litomosoides sigmodontis.

In one embodiment said allergic and/ or autoimmune disease is selected from the group comprising allergic diseases of the respiratory organs, such as asthma, hay fever, allergic sinusitis, allergic rhinitis, of the gastrointestinal system, such as food allergies, of the skin, such as atopic dermatitis, systemic allergic diseases, such as anaphylactic reactions, autoimmune diseases of the joints and/or skin and/or internal organs, such as rheumatoid arthritis, psoriasis, lupus erythematosus, multiple sclerosis, and inflammatory bowel diseases, such as colitis ulcerosa and Crohn's Disease.

Prefebly, said allergic disease is asthma or hay fever. In one embodiment, said inflammatory bowel disease is colitis, preferably colitis ulcerosa. The term "colitis", as used herein, is meant to include both acute and chronic colitis.

In one embodiment said cystatin has a sequence selected from the group comprising SEQ ID NO: SEQ ID NO:1 (Acanthocheilonema viteae cystatin L43053), SEQ ID NO: 2 (Onchocerca volvulus cystatin M37105), SEQ ID NO:3 (Brugia malayi cystatin AF 177193_1) and sequences that are 70% identical, preferably 80% identical, more preferably 90% identical and, most preferably, 95, 96, 97, 98 and 99% identical to any of the foregoing.

In one embodiment said cystatin is recombinant cystatin and has been produced by a procaryotic or eucaryotic expression system.

In one embodiment said disease is associated with an increased count of eosinophil blood cells and/or with an increased level of IgE, when compared with a patient not having said disease, and said medicament, upon its administration to said patient, leads to a reduction of said increased count of eosinophil blood cells and/or to a reduction of said increased level of IgE, preferably to a count of eosinophil blood cells and/or to a level of IgE of a healthy individual.

Preferably, said increased count of eosinophil blood cells is > 4% of all white blood cells of a patient or > 360/µl total number of eosinophil blood cells in peripheral blood of a patient, and said increased level of IgE is > 100 kU/l serum level in an adult patient.

In one embodiment said cystatin is administered to said patient as a protein.

In another embodiment said cystatin is administered to said patient as a nucleic acid encoding said cystatin.

In one embodiment said cystatin is administered systemically to said patient, preferably by injection, inhalation and or other incorporation such as ingestion.

In one embodiment said cystatin is administered to said patient intranasally, intrapulmonarily, intraperitoneally, intrathecally, intralesionally, subcutaneously and/or intramuscularly.

In a preferred embodiment said cystatin is administered in combination with another drug selected from the group of anti-inflammatory drugs, such as corticosteroids, non-steroidal anti-inflammatory drugs and/or anti-histamines.

In one embodiment said patient is a mammal, preferably a human being. In another embodiment, said patient is an animal. Preferably said patient is a canine animal, preferably a dog, or is a feline animal, preferably a cat, or is a rodent, preferably a mouse.

In one embodiment said cystatin is used to bind to CD36 receptor.

The objects of the present invention are also solved by a method of screening for a candidate drug useful for the prevention and/or treatment of an allergic and/or autoimmune disease comprising the following steps:
- providing a first group of cells of a type expressing CD36-receptor,
- exposing said cells to a cystatin derived from a nematode, said cystatin and said nematode being defined as in any of claims 1-21,
- detecting and quantitating, as a first signal, the extent of binding between said cystatin and said CD36 receptor,
- providing a second group of cells of the same type as the first group of cells,
- exposing said second group of cells to a candidate compound,
- detecting and quantitating, as a second signal, the extent of binding between said candidate compound and said CD36 receptor,
- comparing said first signal with said second signal, and
- identifying said candidate compound as a candidate drug for the prevention and/or treatment of an allergic and/or autoimmune disease, if the extent of binding quantitated by said second signal is equal to or greater than the extent of binding quantitated by said first signal.

The objects of the present invention are also solved by a method of treatment or method of prevention of an allergic and/or autoimmune disease in a patient, comprising: administering a cystatin derived from a nematode, to a patient in need thereof. The allergic and/or autoimmune diseases are preferably as defined above. The patient and the cystatin are preferably as defined above. The administration is performed as defined above.

Preferably said cystatin is administered to said patient as a protein.

In another embodiment said cystatin is administered to said patient as a nucleic acid encoding said cystatin.

In one embodiment said cystatin is administered systemically to said patient, preferably by injection, inhalation and or other incorporation such as ingestion.

In one embodiment said cystatin is administered to said patient intranasally, intrapulmonarily, intraperitoneally, intrathecally, intralesionally, subcutaneously and/or intramuscularly.

In a preferred embodiment said cystatin is administered in combination with another drug selected from the group of anti-inflammatory drugs, such as corticosteroids, non-steroidal anti-inflammatory drugs and/or anti-histamines.

As used herein, the term "cystatin" refers to members of a super family of inhibitors of cysteine proteases

A "parasitic nematode" is a nematode that exploits a host organism for meeting its own requirements. This may involve a life of the nematode within the tissue of a host for parts or the entire life-span of the nematode. In preferred embodiments, the host of such parasitic nematode is human.

A sequence that has x% identity to another sequence is a sequence in which x% residues on their respective positions are identical when optimally aligned.

The term "a cystatin derived from a nematode" as used herein, is meant to refer to any cystatin that has the sequence of a cystatin occurring in a nematode. The term is not limited to a specific production method and may include isolation of the cystatin from the nematode or production of the cystatin by other methods, such as recombinant techniques or chemical synthesis. The term "a cystatin derived from a nematode" is also meant to include proteins that have retained a cystatin function despite their amino acid sequence having been mutated in one or several positions by substitutions, insertions or deletions. Techniques for producing such mutated cystatins which nevertheless can be considered as being "derived from a nematode" have been described in, for example, "Proteins, Structures and Molecular Properties" by Thomas E. Creighton, second edition, W. H. Freeman and Co., New York, and are known to someone skilled in the art.

The term "said cystatin is administered in combination with another drug" is meant to include any situation wherein said cystatin is administered concomitantly with, before or after administration of such another drug. The other drug and cystatin may be in the same dosage unit, or they may be administered in separate dosage units. They may be administered by the same route or different routes.

The present inventors have surprisingly found that cystatins from parasitic nematodes are capable of suppressing inflammatory immune reactions in their host. In a number of experiments, the present inventors have been able to demonstrate that cystatins of nematodes can suppress immune reactions and are therefore capable of for example inhibiting the induction of allergic airway hyperreactivity, or the induction of allergic gastrointestinal hyper reactivity. Because of the common etiology between allergic diseases and autoimmune diseases, it can also be reasonably assumed that the cystatins according to the present invention are also useful in the treatment and/or prevention of autoimmune diseases. The inventors could furthermore show that the cystatins according to the present invention have an influence on regulatory T-cells, in that they upregulate and induce regulatory T-cells. Moreover, administration of cystatins according to the present invention leads to a substantial reduction in the count of eosinophil blood cells and a reduction of the levels of allergen-specific immune globulin E (IgE). The administration of cystatins according to the present invention leads to a reduction of these two variables back to approximately "normal" levels, i.e. levels of a healthy individual.

Moreover, the present inventors show that on a molecular basis, the cystatins according to the present invention interact with the CD36-receptor which makes the CD36-receptor a prime target for future drug studies, more specifically in research aimed at finding new therapies for the treatment and/or prevention of allergic diseases and/or autoimmune diseases.

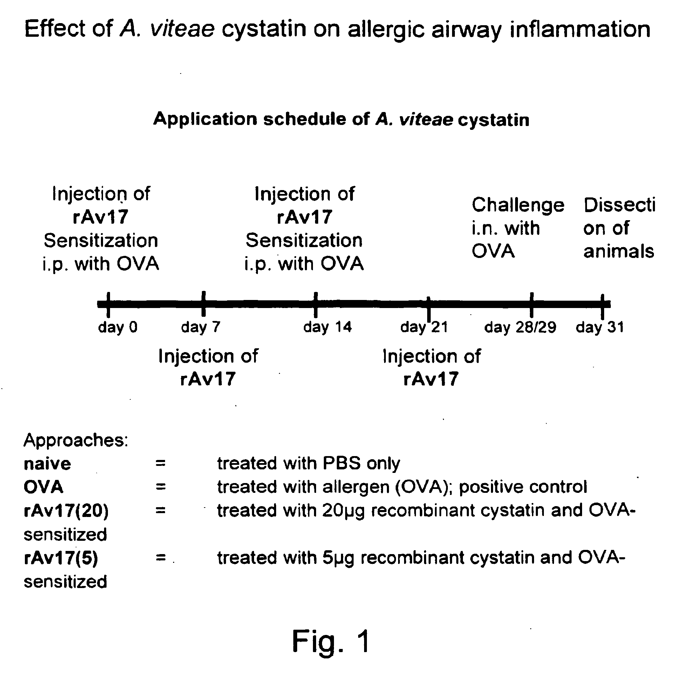

In the following, reference is made to the figures, wherein
figure 1 shows an application schedule of A. viteae cystatin (recombinant Acanthocheilonema viteae cystatin (rAv 17) at two different concentrations (20 µg and 5 µg)),

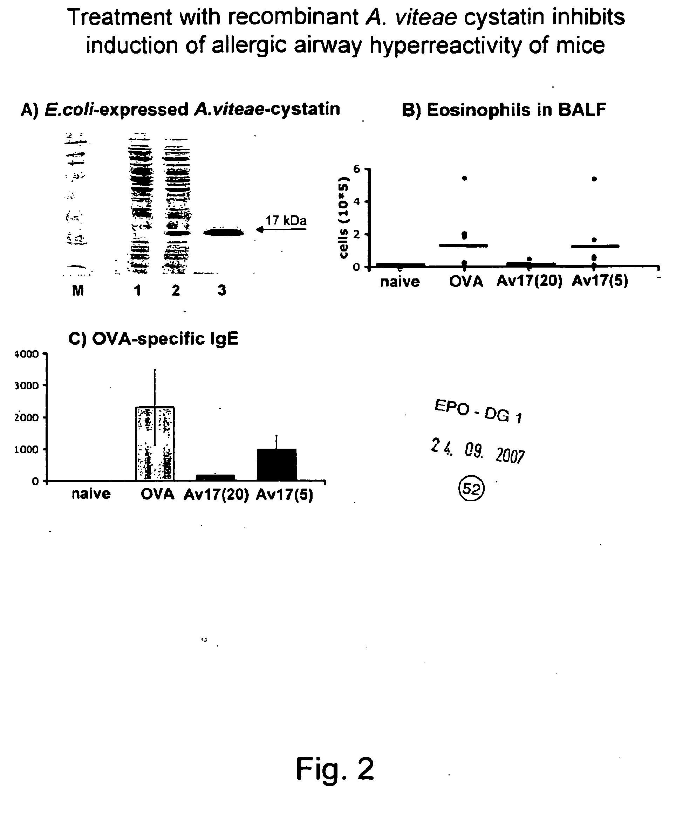

figure 2 shows the influence of A. viteae-cystatin on an animal model for allergic airway inflammation in mice, wherein figure 2a shows an SDS-gel of purified recombinant A. viteae-cystatin, figure 2b shows the numbers of eosinophils in the bronchoalveolar fluid (BALF), figure 2c shows the serum levels of ovalbumine-specific IgE, and figure 2d shows the production of IL-5 in bronchoalveolar fluid, wherein naive means mice treated with PBS (phosphate buffered saline only, OVA means mice treated with ovalbumine, rAv17(20) or (5) means rAv17/OVA treated mice with 20 or 5 µg of rAv17, respectively,

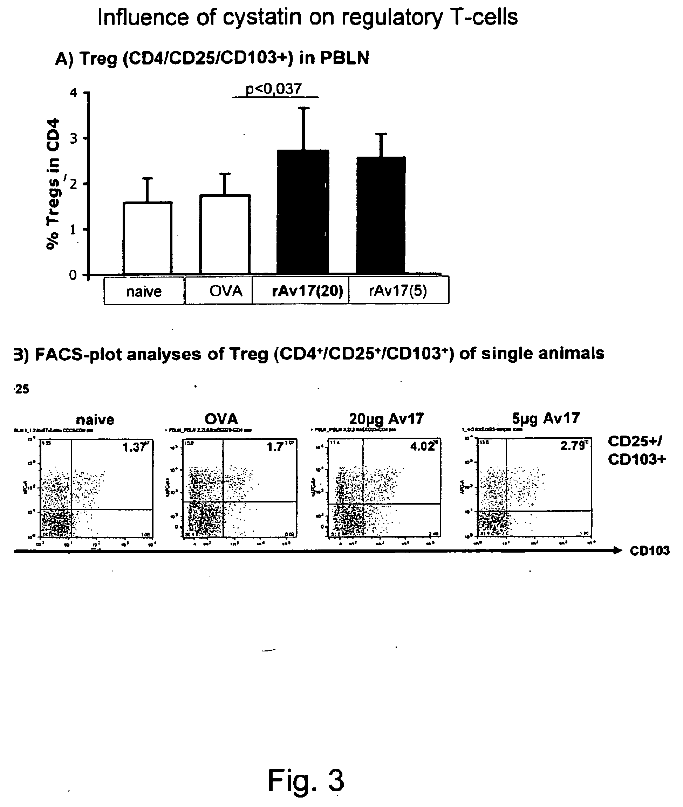

figure 3 shows the influence of cystatins according to the present invention on regulatory T-cells, wherein figure 3a shows the percentage of regulatory T-cells in peribroncheal lymphnode cells (PBLN), and figure 3b) shows FACS-plot analyses of stained cells of a single animal per group; for a legend of figure 3a), see comments to figure 2;

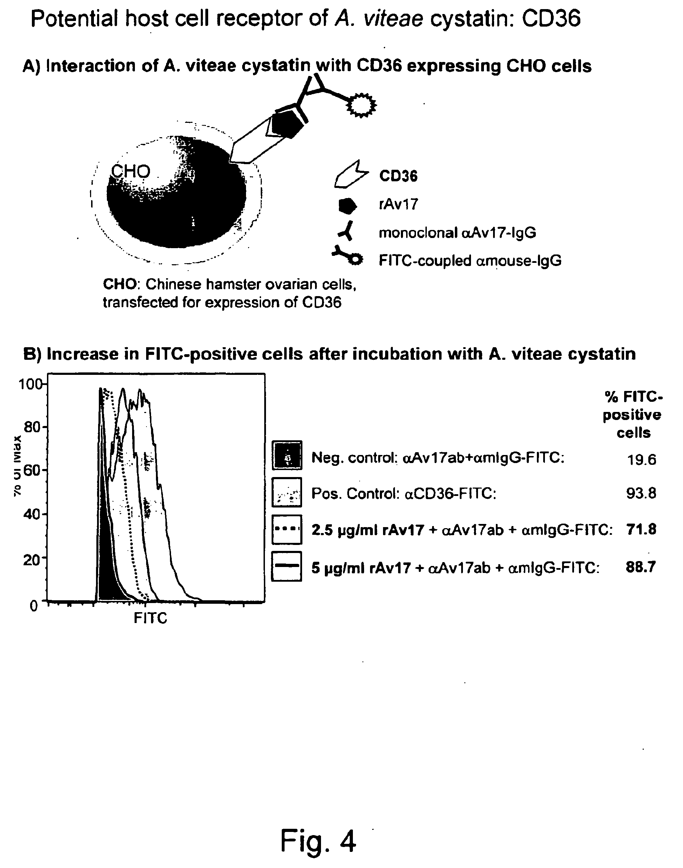

figure 4 shows the interaction of a cystatin according to the present invention with CD36-expressing CHO-cells, wherein figure 4a is a schematic diagram of the assay used, and figure 4b is a representative analysis of the interaction of rAv17 with CD36 in comparison to CD36-negative cells,

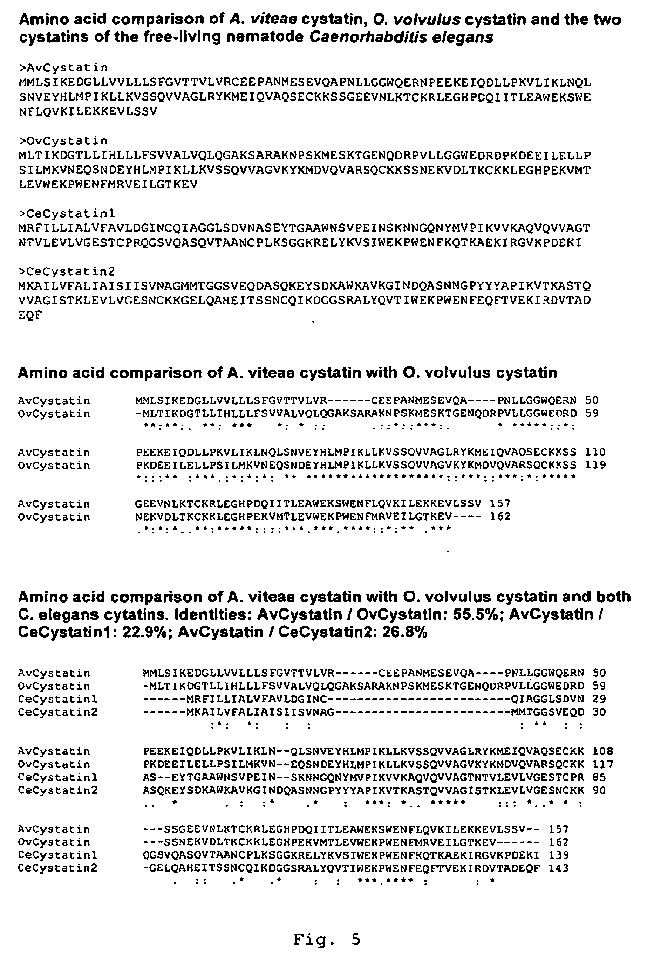

figure 5 shows the sequences of cystatins from A. viteae, O. volvulus and two cystatins from C. elegans,

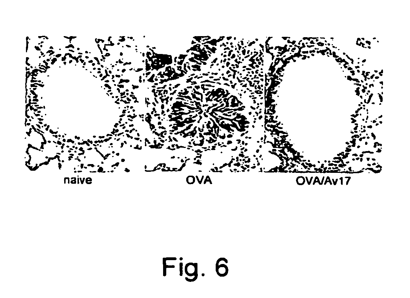

figure 6 shows histological analyses of lung tissue of mice, naïve, treated with OVA and co-treated with OVA/cystatin,

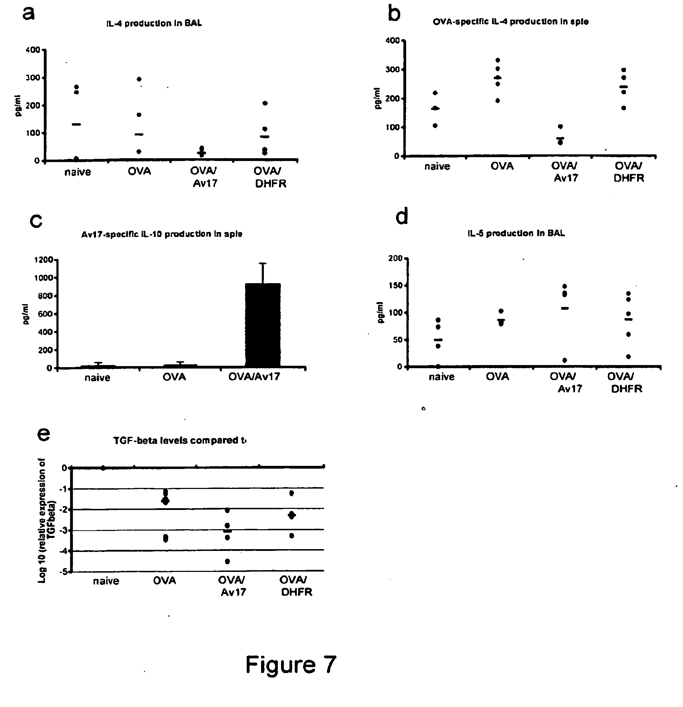

figure 7 shows the production of IL-4 in BALF of mice treated with OVA/cystatin (figure 7 a) spleen cells of mice restimulated with OVA (figure 7b), figure 7c shows the production IL-10 in spleen cells, figure 7d shows the levels of IL-5 in BALF and OVA-restimulated spleen cells, and figure 7e shows TGF-beta-level in lung tissue in OVA/cystatin-treated animals in comparison to OVA-treated animals;

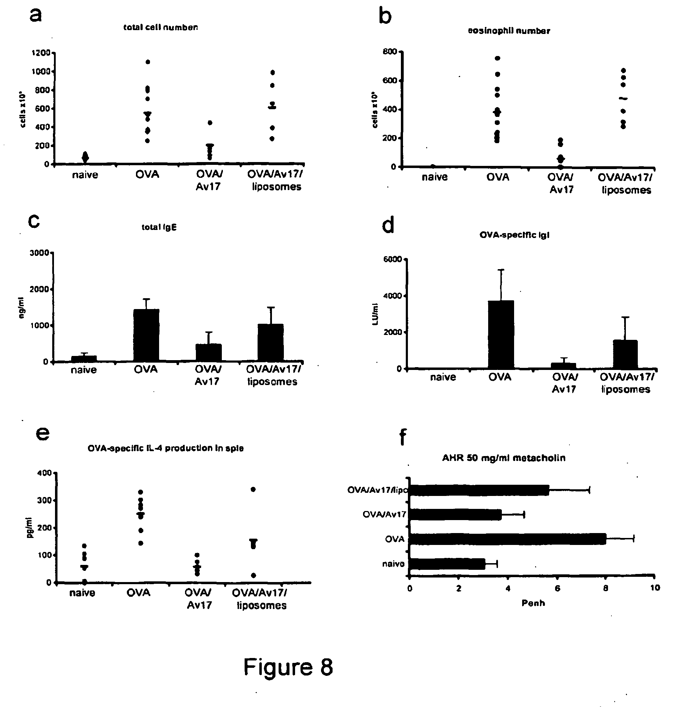

figure 8 shows the effect of a depletion of macrophages of OVA/cystatin-treated mice with respect to the total cell number (figure 8a) and eosinophils (figure 8b), the effect of a depletion of macrophages on the levels of total IgE (figure 8c) and OVA-specific IgE (figure 8d) and of OVA-specific IL-4 in spleen (in figure 8e); figure 8f shows the allergic airway hyperreactivity (AHR) in response to a macrophage-depletion for OVA/cystatin-treated mice;

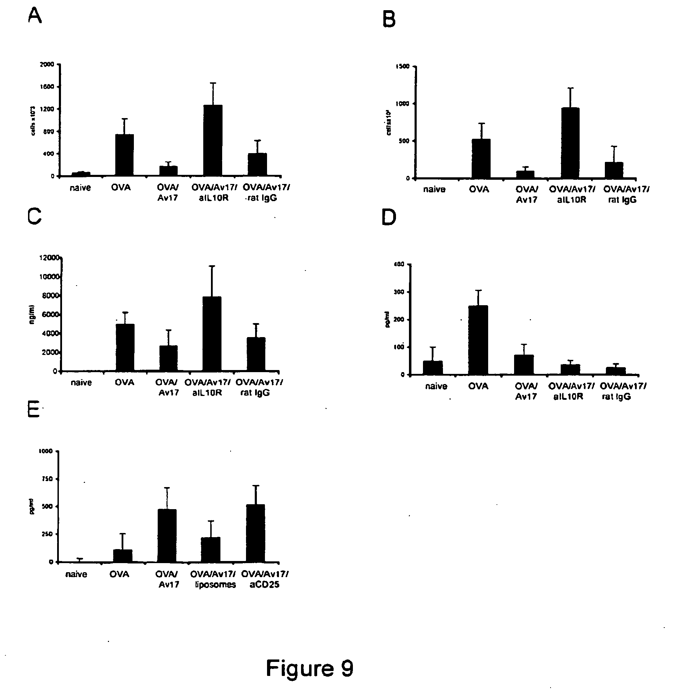

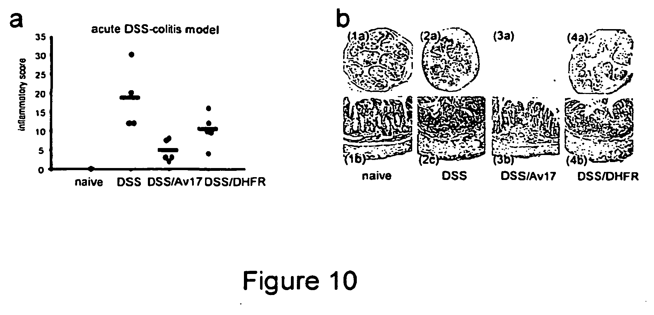

figure 9 shows the effect of a blockage of the IL-10-Rezeptor (IL-10R) by application of an appropriate antibody on the total number of cells (figure 9a), the eosinophil number (figure 9b), the OVA-specific IgE-production (figure 9c) and the OVA-specific IL-4 production; more specifically Figure 9 shows that a suppression of allergic responses by filarial cystatin is dependent on IL-10. Mice were sensitized with ovalbumin (OVA) and treated three times with cystatin (20 µg/ml) and with anti-IL-10R antibodies (500 µg per animal per treatment) after sensitization and prior to challenge with OVA (pre-challenge model). Total cell numbers (A); eosinophil numbers (B); levels of OVA-specific serum IgE (C); OVA-specific IL-4 production of spleen cells (D). (E) IL-10 production of spleen cells after stimulation with Av17. Naive: PBS-treated mice; OVA: ovalbumin-treated mice; OVA/Av17: ovalbumin and filarial cystatin (Av17)-treated mice; OVA/Av 17/aIL-10R: mice treated with ovalbumin and Av17 + anti-IL-10 receptor antibodies; OVA/Av17/rat IgG: mice treated with ovalbumin and Av17 + isotype-matched control antibodies; OVA/Av17/liposomes: ovalbumin and Av17-treated mice in which macrophages were depleted; OVA/Av17/aCD2 ovalbumin and Av17-treated mice in which T_{reg} cells were depleted figure 10 shows the effect of filarial cystatin in an acute DSS-colitis model (inflammatory score-figure 10a) (histological analysis-figure 10b) Colon histology (B): Appearance of the colon in a healthy mouse (1), mouse receiving DSS and the protein application buffer (2), mouse receiving DSS and rAv17 (3) and control animal treated with DSS and the recombinant control protein rDHFR (4). Note extensive epithelial damage with loss of crypts, erosions, dense inflammatory cell infiltrations, goblet cell depletion and thickening of colon wall (2b and 4b) as compared to only focal and superficial erosions associated with less inflammatory cell infiltrations (3b). Magnification × 40 (upper row of a) and × 200 (lower row of b).

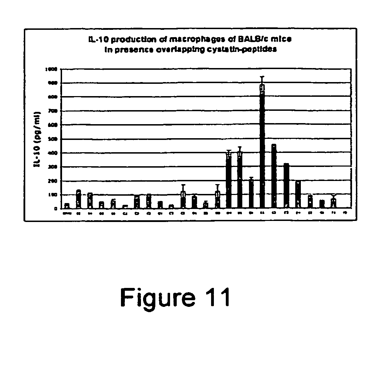

Figure 11 shows the result of a screening of a peptide library to identify specific parasite motifs which are involved in the induction of cytokines.

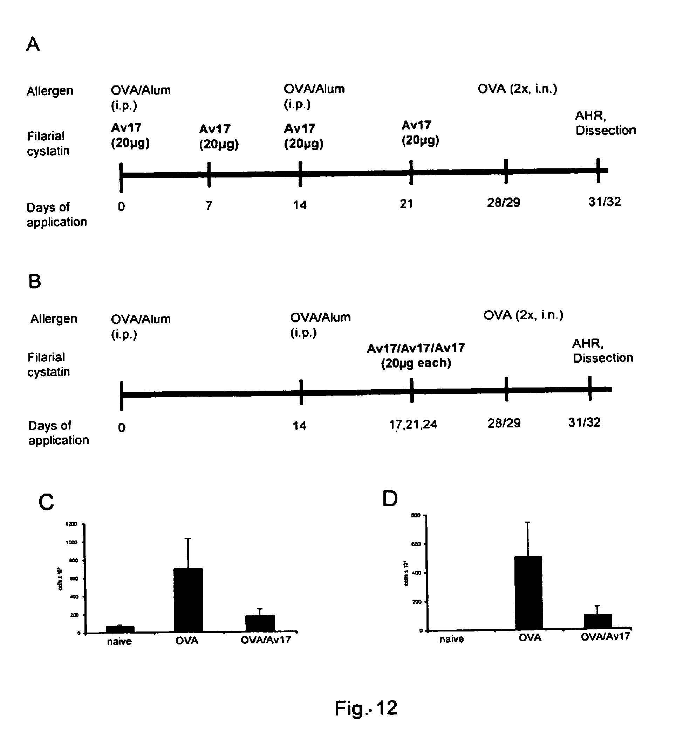

Figure 12 shows a scheme of airway hyperreactivity models. Scheme of the preventive (A) and the pre-challenge (B) model of airway hyperreactivity. Filarial cystatin interferes with the recruitment of total cells (C) and eosinophils (D) in the pre-challenge model. Naive: PBS-treated mice; OVA: ovalbumin-treated mice; OVA/Av17: ovalbumin and filarial cystatin (Av17)-treated mice.

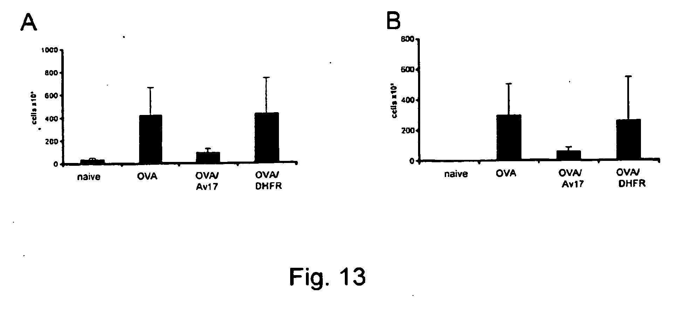

Figure 13 shows that filarial cystatin interferes with cellular recruitment and mucus production in the lung. Total cell numbers (A) and eosinophil numbers (B) in the BALF of mice treated with filarial cystatin (Av17) or a recombinant control protein dehydrofolate reductase (DHFR) observed in the preventive model. Naive: PBS-treated mice; OVA: ovalbumin-treated mice; OVA/Av17: ovalbumin and filarial cystatin (Av17)-treated mice; OVA/DHFR: ovalbumin and DHFR-treated mice.

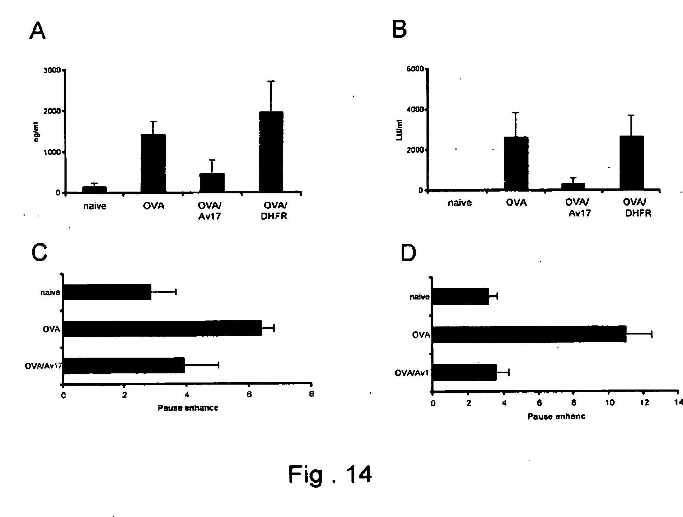

Figure 14 shows that filarial cystatin reduces serum IgE levels and airway hyperreactivity. Total IgE levels (A) and ovalbumin (OVA)-specific IgE concentrations (B) in sera of filarial cystatin-treated mice observed in the preventive model; airway hyperreactivity after application of 50 mg/ml metacholine to mice treated with filarial cystatin in the preventive model (C) and in the pre-challenge model (D). Naive:PBS-treated mice; OVA: ovalbumin-treated mice; OVA/Av17: ovalbumin and filarial cystatin (Av17)-treated mice; OVA/DHFR: ovalbumin and DHFR-treated mice.

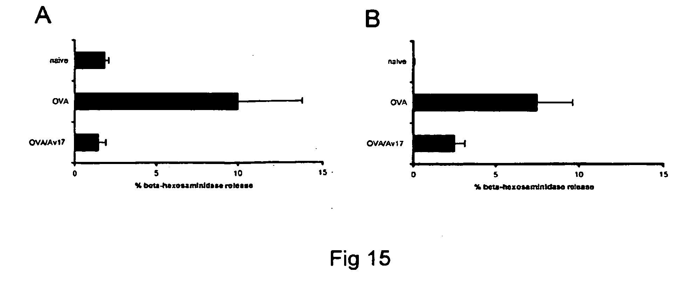

Figure 15 shows a reduced capacity of sera of filarial cystatin-treated mice to induce cellular degranulation. Cystatin treatment in preventive model (A), cystatin treatment in pre-challenge model (B). Rat basophile leukemia (RBL) cells were sensitized with sera of differently treated mice according to Hartmann et al. 2003. Degranulation of cells was subsequently stimulated by addition of ovalbumin (50µg/ml). Naive: sera of PBS-treated mice; OVA: sera of ovalbumin-treated mice; OVA/Av17: sera of ovalbumin and filarial cystatin (Av17)-treated mice.

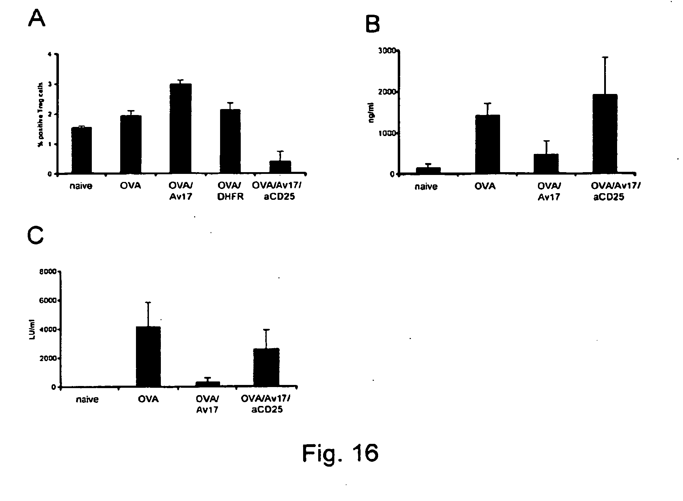

Figure 16 shows that depletion of CD25-positive cells partly reversed the immunomodulation exerted by filarial cystatin. Mice were sensitized with ovalbumin (OVA) and treated with cystatin during the phase of sensitization (preventive model); T_{reg} cells were depleted by application of anti-CD25 antibodies five days prior to challenge. Numbers of T_{reg} cells (CD4⁺CD25⁺CD103⁺) in PBLN (A); levels of total IgE (B); levels of OVA-specific serum IgE (C). PBLN: peribronchial lymph node cells; naive: PBS-treated mice; OVA: ovalbumin-treated mice; OVA/Av17: ovalbumin and filarial cystatin (Av17)-treated mice; OVA/Av17/aCD25: ovalbumin and Av17-treated mice in which T_{reg} cells were depleted by application of anti-CD25 antibodies.

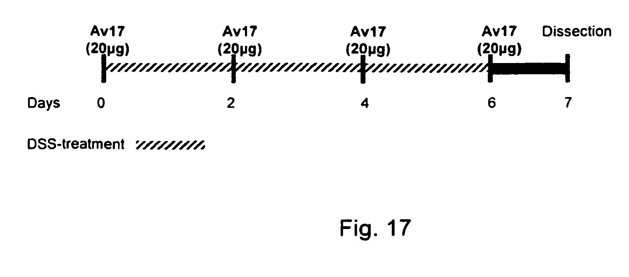

Figure 17 shows a scheme of induction of acute colitis by application of DSS. Av17: filarial cystatin; DSS: dextran sodium sulphate.

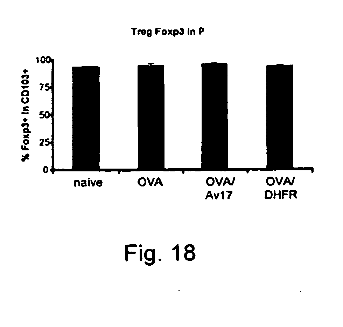

Figure 18 shows the percentage of Foxp3 positive cells within regulatory T cells (CD4⁺CD25⁺CD103⁺) in PBLNCs. Mice were sensitized with ovalbumin (OVA) and treated with cystatin or the recombinant control protein dehydrofolate reductase (DHFR) during the phase of sensitization (preventive model). PBLNC: peribronchial lymph node cells; naive: PBS-treated mice; OVA: ovalbumin-treated mice; OVA/Av17: ovalbumin and filarial cystatin (Av17)-treated mice; OVA/Av 17/DHFR: ovalbumin and DHFR-treated mice.

The present inventors use a so-called "preventive" and "pre-challenge" model of airway hyper reactivity. In the preventive model, the cystatin is administered in weakly intervals during the sensitization, whereas in the pre-challenge model, the cystatin is administered after sensitization prior to airway allergen challenges.

Allergic airway hyperreactivity (AHR) using metacholine is defined as bronchoconstriction after inhalation of metacholine, and is measured as enhancement of the pause between breaths ("enhanced pause").

Moreover, reference is made to the following sequences, wherein SEQ ID NO:1 is the protein sequence of cystatin of Acanthocheilonema viteae (cystatin L43053),
SEQ ID NO:2 is the protein sequence of cystatin of Onchocerca volvulus (cystatin M37105), and SEQ ID NO:3 is the protein sequence of cystatin of Brugia malayi.

In the following, reference is made to the examples which are given to illustrate, not to limit the present invention.

### Examples

### Example 1

### Production of Cystatin from Nematodes

The cDNAs of *A. viteae*-cystatin (Av17, Hartmann, S., Kyewsky, B., Sonnenburg, B. & Lucius, R. (1997) A filarial cysteine protease inhibitor downregulates T cell proliferation and enhances IL-10 production. Eur. J. Immunol. 27, 2253 - 2260.), and of *O. volvulus*-cystatin (Ov17, Lustigman S, Brotman B, Huima T, Prince AM, McKerrow JH. (1992) Molecular cloning and characterization of onchocystatin, a cysteine proteinase inhibitor of Onchocerca volvulus. J Biol Chem. 267:17339-46.); Schönemeyer, A., Lucius, R., Sonnenburg, B., Brattig, N., Sabat, R., Schilling, K., Bradley, J. & Hartmann, S. (2001) Modulation of human T cell responses and macrophage functions by onchocystatin, a secreted protein of the filarial nematode Onchocerca volvulus. J. Immunol. 167: 3207 - 3215) without the sequence encoding for the signal peptide were amplified by PCR using primers (Ov17: forward primer: 5'-gttcagttgcaaggagcc-3', reverse primer: 5'tcatacttcttttgttccc3'; Av17: forward primer: 5'gttttggtgcgctgtgaa3', reverse primer: 5'-tcacactgatgagagtac-3') derived from the full-length sequences. The PCR fragments were cloned into a T-overhang vector (pGEM-T Easy Vector Systems; Promega, Madison, WI) and further subcloned into the Eco-RI site of an expression vector yielding polypeptides with a leader of 6 histidines (pET-28 System; Novagen, Madison, USA). The plasmids were transformed into competent *E. coli* BL21 cells. Screening of transformants for expression was carried out by analysis of bacterial protein after induction with isopropylgalactoside (IPTG). The cell pellet of a 1.5 ml culture was analysed by SDS-PAGE, followed by staining with Coomassie blue. The recombinant protein was purified from the E. coli lysate using a glycerol-PBS-buffer (phosphate buffer saline (PBS) with 10% glycerol) by means of affinity chromatography using a nickel-NTA-column, and subsequently eluted from the column by a pH-change (from pH6 to pH5 to pH3). Subsequently, the eluted protein was dialyzed against PBS/0.05% Triton and sterile filtered.

### Example 2

### Sequence comparison

Figure 5 shows various cystatin sequences and an alignment between cystatin from Acanthocheilonerna viteae and cystatin of Onchocerca volvulus, and a further alignment of the two aforementioned cystatins together with two cystatins (I + II) from the free-living nematode Caenorhabditis elegans. The identity between the first two cystatins is 55.5%, the identity between cystatin from Acanthocheilonema viteae and the two cystatins (I + II) from Caenorhabditis elegans is 22.9% and 26.8%, respectively. The identity between the cystatin of O. volvulus and cystatins I and II from C. elegans is 31.0% and 31.6%, respectively.

### Example 3

### Animal model for allergic airway inflammation and colitis

### Airway inflammation model

The following animal model served as a model for asthma with a severe airway inflammation: BALB/c-mice were sensitized twice using ovalbumin (OVA) (grade VI: Sigma Deisenhofen, Germany) (20 µg/200 µl PBS) in alum (2 mg). Fourteen days after the second sensitization using OVA, the mice were provoked intranasally using 100 pg OVA in 50 µl PBS on two subsequent days. Two days after the airway provocation, the function of the lungs was examined in vivo. One day later, the mice were sacrificed and a bronchoalveolar wash (BAL) was performed (Stock et al., 2004, European Journal of Immunology; 34:1817-1827). Subsequently, the lymphatic tissues were isolated. With respect to the administration of cystatin, the recombinantly produced protein (20 µg or 5 µg) was administered four times at an interval of one week, starting with the first sensitisation two hours prior to administration of the allergen (OVA). The nematode cystatin was administered intraperitoneally. At the time of airway provocation, no nematode cystatin was administered. More specifically, female BALB/c mice (Harlan-Winkelmann, Bachem, Germany) were sensitized twice (day 0 and day 14) intraperitoneally with 20 µg ovalbumin (grade VI, Sigma-Aldrich, Steinheim, Germany) emulsified in 2 mg of aluminium hydroxide (Imject^{®}Alum, Pierce, Rockford, USA) as adjuvant in a total volume of 200 µl. On days 28 and 29, mice were challenged intranasally with 50 µg ovalbumin in 50 µl PBS. Airway responsiveness was measured via whole-body plethysmography on day 31 in unrestrained mice after challenge with increasing doses of metacholine (Sigma), as described in Witzenrath et al., 2006, Am. J. Physiol. Lung Cell Mol. Physiol., 291, 466-472. Recombinant *A. viteae* cystatin (20 pg) or the same amount of control protein (both proteins applied without adjuvant) in PBS was injected intraperitoneally four times in weekly intervals during the sensitization (two hours before ovalbumin i.p. injection), or three times after sensitization prior to airway allergen challenges. Naive control animals were treated with aluminium hydroxide in PBS as a control for the sensitization procedure and these animals were challenged with PBS intranasally instead of ovalbumin.

The following parameters were determined:

Measurement of lung function: The lung function of the mice was measured using a provocation by inhalation with a rising dosage of bronchostringent metacholin, and was subsequently determined in a full body plethysmograph. The pressure difference between the plethysmograph chamber containing the animal and a reference chamber was measured. The pressure difference during the respiratory cycle of the animal can be indicated as enhanced pause ("Penh" or "enhanced pause") using mathematical fomulae and can be taken as an index for respiratory constriction amongst the experimental animals (Hamelmann et al., 1997 Am. J. Respir. Crit. Care. Met.: 156:766-775).

Quantification of cells in bronchoalveolar fluid: Bronchoalveolar lavage was done twice by injecting 0,8ml PBS + protease inhibitors (complete^{™} Mini, Boehringer Mannheim Germany) into the lungs of each animal. The first lavage was centrifuged 10 minutes at 2200rpm-(320g) at 4°C and the supernatant was removed and stored at -20°C for subsequent cytokine analysis. The second lavage was centrifuged at RT (2200rpm, 10min) and the cell pellets of both lavages were pooled and resuspended in 1ml PBS. 100µl of the cell suspension was centrifuged on a glass slide (using a cytospin centrifuge, 10min, 800rpm) creating a cell monolayer which was stained in fixing solution, eosin and thiazo-dye. Subsequently, the number of eosinophils, macrophages, lymphocytes and neutrophils was determined using histological standard protocols according to the manufacturers' instructions (Fisher Diagnostic Scientific, Schwerte, Germany).

Quantification of regulatory T-cells in peribroncheal lymph nodes and their cytokine production; using specific surface markers (CD4, CD25, CD103) and the expression of the transcription factor Foxp3, regulatory T-cells were determined using FACS (Lehmann et al, PNAS USA, 2002; 99:13031-13036). The production of cytokines (IL-4, IL-5, IL-10) of the cells of the bronchoalveolar wash and of the spleen was determined using ELISA, performed according to the manufacturers' instructions of BDE Biosciences using OptEIA™-Kits. More specifically, regulatory T cells in peribronchial lymph nodes were characterized by expression of the surface markers CD4, CD25 and CD103 (49) and the transcription factor Foxp3. Cells were washed in cold PBS/0.2% BSA and stained 15 min on ice with anti-CD4-FITC (BD Biosciences, clone RM4-5), anti-CD25-APC (BD Biosciences, clone PC61), anti-CD103-Bio (gift from Alexander Scheffold, DRFZ, Berlin, clone M290) and streptavidin-PECy7 (BD Biosciences). Foxp3-staining was performed with the PE-anti-mouse Foxp3 staining kit purchased from eBioscience (San Diego, USA, clone FJK-16s) according to the manufacturer's instructions. Nonspecific surface binding of antibodies was prevented by addition of anti-mouse FcyR (clone 2.4G2, gift from Alexander Scheffold, DRFZ, Berlin). Nonspecific intracellular binding during staining of Foxp3 was inhibited by blocking with whole rat IgG (Jackson Laboratories, Cambridgeshire, UK). FACS analysis was performed on LSR II (BD Biosciences).

Total IgE and allergen-specific IgE-production: Total IgE-concentration as well as the OVA-specific IgE-concentration in serum was determined using ELISA. For determining the total IgE-titer, a sandwich ELISA was performed using an anti-IgE-antibody as a catcher-antibody, the sera of the experimental animals (diluted 1:100 in PBS/Tween) and biotinylated anti-IgE antibody as detection antibody. After incubation with strepavidin peroxidase (1:10,000), the reaction of the substrate TMB was photometrically detected at 460 nm. For determining the concentration, a commercially available IgE standard was used. The determination of the allergen-specific IgE was performed using the same method, however, the sera were diluted 1:2 and 1:10 and incubated using biotinylated allergen (50µl of 3µg/ml ovalbumine) as detection molecule. Streptavidinperoxidase (1:10,000) and TMB were used equivalently.

Cytokine production of cystatin treated mice. Splenic mononuclear cells (MNCs) were isolated by density gradient centrifugation (Lympholyte-M, Cedarlane Laboratories, Hornby, Ontario, Canada) and cultured (5x10⁵ cells/ well) in RPMI 1640 with penicillin, streptomycin, L-glutamin and 10% FCS (Hyclone) for 72 hours in the presence of 50 pg/ml ovalbumin or 10 µg/ml Av17 or 10 µg/ml DHFR. Cell culture supernatants were stored at -20°C until performance of cytokine ELISA (IL-4, IL-5, IL-10 BD OptEIA; TGF-beta R&D). Cytokines produced by BAL cells were analyzed the same way. Real time PCR to analyse the expression level of TGF-ß in lung tissue was performed using the 7300 Real-Time PCR System (Applied Biosystems, New Jersey, USA) and TaqMan reagents (TGF-ß primer and probe: Mm 00441729_g1, Applied Biosystems; housekeeping gene GAPDH (glyceraldehyde-3-phosphate dehydrogenase): Mm 99999915_g1, Applied Biosystems). PCR conditions: 95°C for 10 min, followed by 40 cycles of 95°C for 15 s and 60°C for 1 min. Expression of TGF-ß relative to the endogenous GAPDH control was determined using DDC, method as described in Livak et al., 2001, Methods, 25, 402-408.

Depletion of macrophages. Macrophages were depleted by application of clodronate liposomes intranasally and intraperitoneally (100µl /application). Clodronate liposomes were prepared as described elsewhere (Van Roijen N 1994, J. Immunol. Methods., 174, 83-93). In brief, phosphatidylcholin and cholesterol was vacuum evaporated and clodronate filled multilamellar vesicles emerged by gentle shaking under nitrogen condition. The vesicles were kept under nitrogen until washing with steril PBS twice and resuspension in the same puffer. Macrophage depletion was analyzed by flowcytometry or histological cell differentiation, respectively. T_{reg} cells were depleted by i.p. application of 100 µg anti-CD25 antibodies (clone PC61, gift from Alexander Scheffold, DRFZ, Berlin) 5 days prior to challenge. T_{reg} depletion was confirmed by flow cytometry. Anti-IL-10 receptor antibodies (clone 1B1, gift from Kirsten Falk, MDC, Berlin) were applied three times i.p., 500 µg each time along with filarial cystatin. An isotype-matched control antibody from rat served as control (Sigma/Aldrich, St. Louis, USA).

Colitis model. 7-8 week old male C57BL/6 mice weighing 18-20 g where used for experiments. Animals were housed at 22°C under controlled SPF conditions. All experiments were performed in accordance with the German legislation on the protection of animals. Mice were fed with sterile drinking water containing 2.5% dextran sodium sulfate (DSS, mol. wt 40 000, ICN, Eschwege, Germany) for 7 days. Control animals were fed tap water without DSS. Mice were treated intraperitoneally four times over the 7-day DSS feeding period with 20µg rAv17 or the control protein rDHFR in 200µl buffer (day 1, 3, 5 and 7). An additional group received DSS and was sham-treated with the protein application buffer. Appearance of feces and weight loss were monitored daily. In the acute model, animals were killed on day 8 by cervical dislocation, while animals in the chronic model passed through two additional DSS/protein-treatment cycles intermitted by 5 days of recovery on normal drinking water. On the day of dissection, the colon was resected between the ileocecal junction and the proximal rectum. The colon was placed on a non-absorbend surface and measured with a ruler. The entire colon was divided into three segments (proximal, middle and distal) and part of each segment was fixed in 10% neutral buffered formalin. After fixation, specimens were embedded in paraffin, cut into 7 µm sections and stained with hematoxilin and eosin to asses the degree of inflammation. A score of 0-8 (8 being most severe) was assigned for epithelial loss and inflammatory infiltration. Mice were scored individually, with each value representing the mean score and three sections of the distal third of the colon.

Induction of IL-10 by cystatin peptides. Peritoneal excudate cells of male BALB/c mice were harvested by washing the peritoneal cavity 3x with ice cold RPMI 1640 with penicillin, streptomycin, L-glutamin. Cells were plated in flat bottom plates (2x10⁵/well) and led adhere for 2 hours at 37°C. After washing the cells were incubated with 1µg/ml of the cystatin-derived peptides for 24h in a final volume of 200µl. Cell culture supernatants were analyzed for IL-10 by ELISA (BD OptEIA).

Statistical analysis was performed with the Wilcoxon test. Data are presented as means ± standard deviation. Values of p<0.05 were considered as significant.

### Example 4

### Results of experiments

A treatment with recombinant A. viteae cystatin in a mouse model of allergic airway hyper reactivity demonstrates the influence of *A*. *viteae*-cystatin on allergic airway inflammation in mice (Figure 2). 5 mice per group were sensitized with ovalbumin (OVA) and treated with A. viteae-cystatin (Av17). Mice were sensitized twice with 20pg ovalbumin in alum (i.p.) and challenged 14 days after the second sensitization with 50µg ovalbumin in PBS (i.n.). 20 µg cystatin or 5µg cystatin were applied 4 times in weekly intervals during the time of sensitization. Sensitization and challenge with OVA led to a significant increase in total cell number reflected by the numbers of eosinophils in the bronchoalveolar fluid. The concurrent treatment with 20µg/ml A. viteae cystatin completely abolished the effect of OVA on eosinophils (Fig. 2b). Such a strong influence could not be seen by a lower dosage of cystatin (5µg/ml).

Treatment with 4 doses of cystatin (20µg each) during sensitization (scheme of preventive model, Fig. 12 A), but not with the irrelevant recombinant control protein murine dihydrofolate reductase (DHFR), significantly reduced the total numbers of cells (p<0.028) in the bronchoalveolar lavage fluid (BALF) to the level of naïve (non sensitized, non challenged) mice (Fig. 13 A). This effect was most pronounced for eosinophils (p<0.05) (Fig. 13 B). Similar results were obtained when cystatin was applied three times after sensitization (p<0.02) (scheme of pre-challenge model and cell numbers in BALF, Fig. 12 B, C, D). Histological analysis of the lung tissue corroborated these data, showing only background levels of cell infiltration within the lung tissue and a nearly absent mucus production in mice co-treated with ovalbumin/cystatin (Fig. 6).

A second feature of an allergic airway inflammation is the significant upregulation of the allergen-specific IgE concentration. Again, as shown in figure 2c, the inventors revealed that sensitization and challenge with OVA significantly induced the production of OVA-specific IgE in sera of mice. However, treatment with cystatin completely reversed the impact on allergen-specific IgE.

Treatment with cystatin also significantly reduced serum levels of ovalbumin-specific IgE (p<0.0002) as well as levels of total IgE (p<0.0003), an effect not seen after injection of the recombinant control protein DHFR (Fig. 14 A, B). This effect was specific to IgE, as serum levels of ovalbumin-specific IgG1 and IgG2a were not significantly altered compared to sensitized and challenged controls (data not shown). The significant inhibition of ovalbumin-specific and total IgE production was accompanied by a reduced capacity of sera from cystatin/ovalbumin-treated mice to induce degranulation of basophils (Fig. 15 A, B). The effects of cystatin on allergen-induced sensitization and airway inflammation were accompanied by a significant reduction (p<0.028) in the development of *in vivo* airway hyperreactivity (AHR) in treated mice in the preventive as well as in the pre-challenge model (Fig. 14 C, D). These data suggest that cystatin interferes with the recruitment of inflammatory cells and with IgE production, thus inhibiting the main features of allergen-induced alterations in this mouse model both during and after sensitization.

Another prominent characteristic of an airway hyperreactivity is the influence of cystatin treatment on cytokines. To determine the mechanisms responsible for reduction of allergic responses, the present inventors analysed the cytokine pattern of experimental animals. BALF of mice treated with OVA/cystatin contained less IL-4 as compared to BALF of animals treated with OVA only, or with OVA/DHFR. This effect was systemic, as spleen cells of mice restimulated with OVA also produced significantly less IL-4 (p<0.015) (**Fig. 7a****, b**). Interestingly they could not determine elevated levels of IL-10 in BALF however, the levels of IL-10 in spleen cells at the time point of dissection were elevated in spleen (p<0.002) in animals that had received their last dose of cystatin 4 days before challenge, but were normal in animals treated earlier (**Fig. 7c**). The levels of IL-5 in BALF and cultures of OVA-restimulated spleen cells were not significantly altered by treatment with cystatin (**Fig. 7d**). TGF-β levels were determined in lung tissue and found to be decreased in the OVA/cystatin-treated group in comparison to the OVA-group measured by real time PCR (**Fig. 7e**). These data are compatible with an overall downregulation of effector molecules of allergic reactions such as eosinophils, IgE and IL-4 by filarial cystatin. Together, these data indicate that repeated treatment with 20 ug cystatin reduces allergic airway inflammation to the level of a healthy individual. Histological analysis of the lung tissue corroborated these data, showing background levels of infiltrating cells within the lung tissue and of mucus production of mice co-treated with OVA/cystatin (see fig. 6).

The panels A - D of figure 2 show the following: A: SDS-gel of purified recombinant *A. viteae*-cystatin; B: Numbers of eosinophils in the bronchoalveolar fluid (BALF). C: Serum levels of OVA-specific IgE. Naive: PBS-treated mice; OVA: ovalbumin-treated mice; Av17: Av17/OVA-treated mice with 20 or 5 µg Av17.

Furthermore, *A. viteae*-cystatin showed an influence on induction of regulatory T cells in PBLNs of mice (Figure 3). Again, 5 mice per group were sensitized with ovalbumin (OVA) and treated with *A. viteae*-cystatin (Av17). Mice were sensitized twice with 20µg ovalbumin in alum (i.p.) and challenged 14 days after the second sensitization with 50µg ovalbumin in PBS (i.n.). 20 µg cystatin or 5µg cystatin were applied 4 times in weekly intervals during the time of sensitization. Regulatory T cells (Tregs) were characterized by staining of their cell surface markers CD25 and CD103 and double positive cells were 98% Foxp3 positive, a transcription factor, which represents another reliable marker for Tregs. The analyses showed that sensitization of mice with OVA and the subsequent challenge with OVA did not lead to an increase in regulatory T cells in comparison to naive mice. But the animals, which were concurrently treated with cystatin showed a significant increase in regulatory T cells. Figure 3a shows the mean values of Tregs of all animals per group (n=5). Figure 3b show a representaive FACS-plot analysis of a single animal. On the upper right panel of each plot is the percentage of cells shown which were double positive for both Treg markers. These data clearly show that the application of cystatin led to an increase in the number of these potent suppressor cells. More specifically, the proportion of T_{reg} cells was significantly elevated in ovalbumin/cystatin-treated animals as compared to ovalbumin-controls (3% versus 1.9%, p<0.05) (Fig. 16 A) and to ovalbumin/DHFR-controls (3% versus 2.1%, (p<0.05) Fig. 16 A). Approximately 94-98% of the T_{reg} cells expressed Foxp3, a reliable marker for T_{reg} cells (Fig. 18). To analyze the role of T_{reg} cells in cystatin-induced immunomodulation the inventors treated sensitized animals with anti-CD25 antibodies two days prior to the first airway allergen challenge, which completely diminished the number of CD25⁺ T_{reg} cells in PBLNCs (Fig. 6 A). In cystatin treated mice with depleted T_{reg} cells the levels of total cells as well as eosinophils, allergen-specific IL-4 production and development of AHR were not significantly altered (data not shown). But production of total IgE (p<0.002) and ovalbumin-specific IgE (p<0.002) was significantly restored compared to cystatin treated animals with unmanipulated T_{reg} cells (Fig. 16 B, C). These data indicate that T_{reg} cells are involved in the cystatin-induced effects on airway hyperreactivity, albeit to a significantly lesser degree than macrophages.

The panels A - B of figure 3 show: A: Percentage of regulatory T cells (CD4+/CD25+/CD103+) in peribroncheal lymph node cells; B: FACS-plot analyses of stained cells of a single animal per group. Naive: PBS-treated mice; OVA: ovalbumin-treated mice; Av17: Av17/OVA-treated mice with 20 or 5 µg Av 17.

### Filarial cystatin targets macrophages

As previous in vitro studies (Schönemeyer et al. 2001, J. Immunol. 2001, Sept. 15; 167 (6) : 3207-3215) have shown that the immunomodulatory effect of cystatin is dependent on macrophages, the inventors studied their relevance by depleting these cells. OVA-sensitized animals, which had received cystatin-treatment during sensitization were selectively depleted of macrophages by application of clodronate liposomes two days prior to challenge with OVA. This treatment led to loss of 95% of macrophages in the BALF and in the peritoneum as analysed by FACS staining of F4/80 positive cells that were negative for CD19 and CD3 (data not shown). Depletion of macrophages of OVA/cystatin-treated mice restored the numbers of total BAL cells (p<0.008) and eosinophils (p<0.002) to the level of the OVA-group **(****Fig. 8a,b****).** The treatment also partly restored the levels of total IgE (p<0.05) and of OVA-specific IgE (p<0.007) in the mouse sera **(****Fig. 8c,d****)** as well as OVA-specific IL-4 in spleen **(****Fig. 8e****).** Likewise, AHR of macrophage-depleted, OVA/cystatin treated mice was almost restored (p<0.04) to levels of animals treated with OVA only **(****Fig. 8f****).** AHR is measured as the pause between breaths after inhalation of metacholine. Together, these results show that macrophages are key cells in the downregulation of allergic responses by cystatin through inhibition of the recruitment of inflammatory cells, lowering of IgE levels as well as partly restoring the IL-4 production.

### Inhibition of allergic responses by cystatin is dependent on IL-10

The present inventors hypothesized that IL-10 might be the key mediator of cystatin-induced immunomodulation and analyzed its influence by application of anti-IL-10 receptor antibodies (anti IL-10R) in the pre-challenge model of OVA-induced airway reactivity (see also figure 12B). Anti-IL-10R was injected 3x along with the treatment of cystatin in-between sensitization and challenge with OVA. Blocking of IL-10R in OVA/cystatin treated animals completely restored the decreased numbers of total cells (p<0.02) **(****Fig. 9a****)**. The effect of IL-10 neutralization was most pronounced for the number of eosinophils in the BALF (p<0.02) **(****Fig. 9b****).** Similarly, the AHR values were increased by application of anti-IL 10R antibodies in comparison to mice treated with OVA only (data not shown) and the OVA-specific IgE production in the OVA/cystatin group was restored (p<0.031) by application of anti-IL10R antibodies **(****Fig. 9c****).** However, the suppressed allergen-specific IL-4 production in the OVA/Av17 animals (p<0.0003) was not reversible by application of anti-IL-10R antibodies **(****Fig. 9d****).** In all cases, application of isotype matched control antibodies had no significant effects. These data indicate that IL-10 is a key mediator of the cystatin-induced immunomodulation, however also IL-10 independent mechanisms like the suppression of allergen-specific IL-4 seem to play a role. As macrophages and T_{reg} cells are both potent sources of IL-10, the inventors asked which of these cells is primarily responsible for the IL-10 induction after treatment with filarial cystatin. Cystatin-treated animals showed significantly increased levels of IL-10 in spleen in comparison to ovalbumin-treated animals (p<0.01) (Fig. 7 C and 9 E). However, after depletion of macrophages, the IL-10 production was significantly decreased in the ovalbumin/cystatin treated animals (p<0.04) (Fig. 9 E), whereas such an effect was not observed in the animals depleted of T_{reg} cells. T_{reg}-depleted mice actually showed a trend of elevated IL-10 values (Fig. 9 E). These data underline the pivotal role of macrophages in the cystatin-induced modulation of allergic airway inflammation and hyperreactivity.

### Filarial cystatin inhibits acute and chronic colitis

To examine whether cystatin inhibits Th1 inflammation, the present inventors also tested the nematode immunomodulator in a murine model of colitis induced by application of 2.5% dextran sodium sulphate (DSS) in the drinking water for 7 days (Figure 17). Intraperitoneal administration of 4 doses of 20µg of filarial cystatin over the period of DSS application revealed a significant reduction (p< 0.03) of the inflammatory score (54%) of the colon as compared to treatment with DSS/DHFR **(****Fig. 10a,b****)**. In a chronic colitis model animals were treated with 4 DSS cycles of one week duration with intervals of one week each, and cystatin was applied 4 times during each DSS cycle. As in the acute colitis model, cystatin resulted in a significant reduction of the inflammatory score as compared to the DSS/DHFR control group of 63%. Hence, cystatin is useful for treatment of both chronic and acute colitis.

### Induction of IL-10 in macrophages by a specific protein domain

The present inventors asked whether cystatin-derived peptides would have the capacity to induce IL-10 production of macrophages and screened a library of 20mer peptides overlapping by three aa, representing the cystatin protein. Incubating the peptide library with murine macrophages harvested from the peritoneal cavity of BALB/c mice identified an IL-10 inducing region of cystatin **(****Fig. 11****)**, reaching from aa 66 to aa 115. The strongest IL-10-production was induced by the peptide aa 81 - 99 that contains two conserved cysteine residues, which are described to form a disulphide bond in the cystatin superfamily (Bode et al. 1988, Janowski et al. 2001). The region between these cysteines does not show homology to known sequences of vertebrate cystatins like human cystatin (25% identity), however within cystatins of other parasitic nematodes homologies of 60% to *Onchocerca volvulus* cystatin, 75% to *Brugia malayi* cystatin and 65% to *Litomosoides sigmodontis* cystatin can be determined, suggesting that a parasite specific motif is involved in induction of IL-10.

In addition, the inventors have evidence for the interaction of *A. viteae*-cystatin with the scavenger receptor CD36(Figue 4). CHO cells (chinese hamster ovaria), which are stabily transfected with CD36 were incubated with recombinant *A. viteae* cystatin (rAv17) in two different concentrations (2.5µg/ml, 5µg/ml). Binding to CD36 was detected by reaction with a monoclonal antibody against cystatin. The receptor/antigen/antibody interaction was subsequently detected by a secondary FITC-labelled antibody. As a positive control a FITC-labelled anti-CD36 antibody was used. The analyses showed that cystatin bound to CD36, which could be detected by the significant increase in fluorescence positive cells from 20% to 71% or 89 % respectively (Fig. 4). An interaction of cystatin with the scavenger receptor CD36 on immune cells such as macrophages could explain its therapeutic potential, as targeting of CD36 results in the production of IL-10 and other anti-inflammatory processes.

### Figure 4 A - B shows:

(A) Schematic diagram of the assay; B) One representative analyses of the interaction of Av17 with CD36 in comparison to CD36 negative cells and the positive control (detection of CD36 on CD36 expressing cells by a FITC-labelled antibody).

Figure 5 shows a sequence comparison of various cystatins. Amino acid comparison of A. viteae and O. volvulus cystatin with the cystatins of the free living nematode C. elegans shows an identity of 56% between the parasitic cystatins in comparison to about 30% to the cystatins of the free-living nematode. Differences in the amino acid sequence of the cystatins of parasitic nematodes in comparison to the free-living nematodes imply functional differences due to specific protein domains.

The features of the present invention disclosed in the specification, the claims and/or in the accompanying drawings, may, both separately, and in any combination thereof, be material for realizing the invention in various forms thereof.

## Claims

1. Cystatin derived from a parasitic nematode for use in a method for the prevention and/or treatment of an allergic and/ or autoimmune disease in a patient.

2. Cystatin for use according to claim 1, **characterized in that** said parasitic nematode is parasitic to humans.

3. Cystatin for use according to claim 1, **characterized in that** said parasitic nematode is parasitic to animals.

4. Cystatin for use according to claim 3, **characterized in that** said parasitic nematode is parasitic to canine animals, preferably dogs, or is parasitic to rodents, preferably mice, or is parasitic to feline animals, preferably cats.

5. Cystatin for use according to any of claims 1-4, **characterized in that** said nematode is selected from the group comprising Onchocerca volvulus, Brugia malayi, Wuchereria bancrofti, Loa loa and Acanthocheilonema viteae, Dirofilaria immitis, Dirofilaria repens, Nippostrongylus brasiliensis and Litomosoides sigmodontis.

6. Cystatin for use according to any of the foregoing claims, **characterized in that** said allergic and/ or autoimmune disease is selected from the group comprising allergic diseases of the respiratory organs, such as asthma, hay fever, allergic sinusitis, allergic rhinitis, of the gastrointestinal system, such as food allergies, of the skin, such as atopic dermatitis, systemic allergic diseases, such as anaphylactic reactions, autoimmune diseases of the joints and/or skin and/or internal organs, such as rheumatoid arthritis, psoriasis, lupus erythematosus, multiple sclerosis, and inflammatory bowel diseases, such as colitis ulcerosa and Crohn's Disease.

7. Cystatin for use according to claim 6, **characterized in that** said allergic disease is asthma or hay fever.

8. Cystatin for use according to claim 6, **characterized in that** said inflammatory bowel disease is colitis, preferably colitis ulcerosa.

9. Cystatin for use according to any of the foregoing claims, **characterized in** said cystatin has a sequence selected from the group comprising SEQ ID NO: 1 (Acanthocheilonema viteae cystatin L43053),
SEQ ID NO: 2 (Onchocerca volvulus cystatin M37105), SEQ ID NO:3 (Brugia malayi cystatin), and
sequences that are 70% identical, preferably 80% identical, more preferably 90% identical and, most preferably, 99% identical to any of the foregoing.

10. Cystatin for use according to any of the foregoing claims, **characterized in that** said cystatin is recombinant cystatin and has been produced by a procaryotic or eucaryotic expression system.

11. Cystatin for use according to any of the foregoing claims, **characterized in that** said disease is associated with an increased count of eosinophil blood cells and/or with an increased level of IgE, when compared with a patient not having said disease, and said medicament, upon its administration to said patient, leads to a reduction of said increased count of eosinophil blood cells and/or to a reduction of said increased level of IgE, preferably to a count of eosinophil blood cells and/or to a level of IgE of a healthy individual.

12. Cystatin for use according to claim 11, **characterized in that** said increased count of eosinophil blood cells is > 4% of all white blood cells of a patient or > 360/µl total number of eosinophil blood cells in peripheral blood of a patient, and said increased level of IgE is > 100 kU/l serum level in an adult patient.

13. Cystatin for use according to any of the foregoing claims, **characterized in that** said cystatin is administered to said patient as a protein.

14. Cystatin for use according to any of claims 1 - 12, **characterized in that** said cystatin is administered to said patient as a nucleic acid encoding said cystatin.

15. Cystatin for use according to any of the foregoing claims, **characterized in that** said cystatin is administered systemically to said patient, preferably by injection, inhalation and/or other incorporation such as ingestion.

16. Cystatin for use according to any of the foregoing claims, **characterized in that** said cystatin is administered to said patient intranasally, intrapulmonarily, intraperitoneally, intrathecally, intralesionally, subcutaneously and/or intramuscularly.

17. Cystatin for use according to any of the foregoing claims, **characterized in that** said cystatin is administered in combination with another drug selected from the group of anti-inflammatory drugs, such as corticosteroids, non-steroidal anti-inflammatory drugs, and/or anti-histamines.

18. Cystatin for use according to any of the foregoing claims, **characterized in that** said patient is a mammal, preferably a human being.

19. Cystatin for use according to any of claims 1-17, **characterized in that** said patient is an animal.

20. Cystatin for use according to claim 19, **characterized in that** said animal is a canine animal, preferably a dog, or is a feline animal, preferably a cat, or is a rodent, preferably a mouse..

21. Cystatin for use according to any of the foregoing claims, **characterized in that** said cystatin is used to bind to CD36 receptor.

22. A method of screening for a candidate drug useful for the prevention and/or treatment of an allergic and/or autoimmune disease comprising the following steps:
- providing a first group of cells of a type expressing CD36-receptor,
- exposing said cells to a cystatin derived from a nematode, said cystatin and said nematode being defined as in any of claims 1-21,
- detecting and quantitating, as a first signal, the extent of binding between said cystatin and said CD36 receptor,
- providing a second group of cells of the same type as the first group of cells,
- exposing said second group of cells to a candidate compound,
- detecting and quantitating, as a second signal, the extent of binding between said candidate compound and said CD36 receptor,
- comparing said first signal with said second signal, and
- identifying said candidate compound as a candidate drug for the prevention and/or treatment of an allergic and/or autoimmune disease, if the extent of binding quantitated by said second signal is equal to or greater than the extent of binding quantitated by said first signal.

## Patentansprüche

1. Cystatin, abgeleitet von einer parasitischen Nematode, zur Verwendung in einem Verfahren zur Verhinderung und/oder Behandlung einer allergischen und/oder Autoimmun-Erkrankung in einem Patienten.

2. Cystatin zur Verwendung nach Anspruch 1, **dadurch gekennzeichnet, dass** die parasitische Nematode für Menschen parasitisch ist.

3. Cystatin zur Verwendung nach Anspruch 1, **dadurch gekennzeichnet, dass** die parasitische Nematode für Tiere parasitisch ist.

4. Cystatin zur Verwendung nach Anspruch 3, **dadurch gekennzeichnet, dass** die parasitische Nematode für hundeartige Tiere, bevorzugt Hunde, parasitisch ist oder für Nagetiere, bevorzugt Mäuse, parasitisch ist, oder für katzenartige Tiere, bevorzugt Katzen, parasitisch ist.

5. Cystatin zur Verwendung nach einem der Ansprüche 1-4, **dadurch gekennzeichnet, dass** die Nematode ausgewählt ist aus der Gruppe, umfassend Onchocerca volvulus, Brugia malayi, Wuchereria bancrofti, Loa loa und Acanthocheilonema viteae, Dirofilaria immitis, Dirofilaria repens, Nippostrongylus brasiliensis und Litomosoides sigmodontis.

6. Cystatin zur Verwendung nach einem der vorangehenden Ansprüche, **dadurch gekennzeichnet, dass** die allergische und/oder Autoimmun-Erkrankung ausgewählt ist aus der Gruppe, umfassend allergische Erkrankungen der Atmungsorgane, wie etwa Asthma, Heuschnupfen, allergische Sinusitits, allergische Rhinitis, Erkrankungen des Gastrointestinalsystems, wie etwa Nahrungsmittelallergien, Erkrankungen der Haut, wie etwa atopische Dermatits, systemische allergische Erkrankungen, wie etwa anaphylaktische Reaktionen, Autoimmun-Erkrankungen der Gelenke und/oder der Haut und/oder der internen Organe, wie etwa rheumatoide Arthritis, Psoriasis, Lupus erythematodes, Multiple Sklerose und entzündliche Darmerkrankungen, wie etwa Colitis Ulcerosa, und Morbus Crohn.

7. Cystatin zur Verwendung nach Anspruch 6, **dadurch gekennzeichnet, dass** die allergische Erkrankung Asthma oder Heuschnupfen ist.

8. Cystatin zur Verwendung nach Anspruch 6, **dadurch gekennzeichnet, dass** die entzündliche Darmerkrankung Colitis ist, bevorzugt Colitis ulcerosa.

9. Cystatin zur Verwendung nach einem der vorangehenden Ansprüche, **dadurch gekennzeichnet, dass** das Cystatin eine Sequenz hat, ausgewählt aus der Gruppe, umfassend SEQ ID NO:1 (Acanthocheilonema viteae Cystatin L43053), SEQ ID NO:2 (Onchocerca volvulus Cystatin M37105), SEQ ID NO: 3 (Brugia malayi Cystatin), und Sequenzen, die 70% identisch, bevorzugt 80% identisch, bevorzugter 90% identisch, und am bevorzugtesten 99% identisch mit einer der vorherigen Sequenzen ist.

10. Cystatin zur Verwendung nach einem der vorangehenden Ansprüche, **dadurch gekennzeichnet, dass** Cystatin rekombinantes Cystatin ist und mittels eines prokaryotischen oder eukaryotischen Expressionssystems erzeugt worden ist.

11. Cystatin zur Verwendung nach einem der vorangehenden Ansprüche, **dadurch gekennzeichnet, dass** die Erkrankung mit einer erhöhten Anzahl an eosinophilen Blutkörperchen und/oder mit einer erhöhten Konzentration an IgE assoziiert ist, im Vergleich mit einem Patienten, der diese Erkrankung nicht hat, und wobei das Medikament, bei seiner Verabreichung an den Patienten, zu einer Verringerung der erhöhten Anzahl an eosinophilen Blutkörperchen und/oder zu einer Verringerung der erhöhten Konzentration an IgE, bevorzugt zu einer Anzahl an eosinophilen Blutkörperchen und/oder zu einer Konzentration von IgE eines gesunden Individuums führt.

12. Cystatin zur Verwendung nach Anspruch 11, **dadurch gekennzeichnet, dass** die erhöhte Anzahl an eosinophilen Blutkörperchen > 4% aller weißen Blutkörperchen eines Patienten oder > 360/µl der Gesamtzahl an eosinophilen Blutkörperchen im Peripherblut eines Patienten ist, und die erhöhte Konzentration an IgE > 100kU/l Serum Konzentration in einem erwachsenen Patienten ist.

13. Cystatin zur Verwendung nach einem der vorangehenden Ansprüche, **dadurch gekennzeichnet, dass** das Cystatin an einen Patienten als ein Protein verabreicht wird.

14. Cystatin zur Verwendung nach einem der Ansprüche 1-12, **dadurch gekennzeichnet, dass** das Cystatin an den Patienten als eine Nukleinsäure, die für das Cystatin kodiert, verabreicht wird.

15. Cystatin zur Verwendung nach einem der vorangehenden Ansprüche, **dadurch gekennzeichnet, dass** das Cystatin systemisch an den Patienten verabreicht wird, bevorzugt mittels Injektion, Inhalation und/oder einer anderen Einnahme, wie etwa Ingestion.

16. Cystatin zur Verwendung nach einem der vorangehenden Ansprüche, **dadurch gekennzeichnet, dass** das Cystatin an den Patienten intranasal, intrapulmonal, intraperitoneal, intrathekal, intraläsional, subkutan und/oder intramuskulär verabreicht wird.

17. Cystatin zur Verwendung nach einem der vorangehenden Ansprüche, **dadurch gekennzeichnet, dass** das Cystatin in Kombination mit einer anderen Arznei verabreicht wird, ausgewählt aus der Gruppe der anti-entzündlichen Arzneien, wie etwa Corticosteroide, nicht-steroide anti-entzündliche Arzneien und/oder Anti-Histamine.

18. Cystatin zur Verwendung nach einem der vorangehenden Ansprüche, **dadurch gekennzeichnet, dass** der Patient ein Säugetier, bevorzugt ein Mensch, ist.

19. Cystatin zur Verwendung nach einem der Ansprüche 1-17, **dadurch gekennzeichnet, dass** der Patient ein Tier ist.

20. Cystatin zur Verwendung nach Anspruch 19, **dadurch gekennzeichnet, dass** das Tier ein hundartiges Tier, bevorzugt ein Hund, oder ein katzenartiges Tier, bevorzugt eine Katze, oder ein Nagetier, bevorzugt eine Maus, ist.

21. Cystatin zur Verwendung nach einem der vorangehenden Ansprüche, **dadurch gekennzeichnet, dass** das Cystatin verwendet wird, um an den CD36-Rezeptor zu binden.

22. Verfahren zum Sreenen auf ein Arzneimittelkandidaten, der zur Verhinderung und/oder Behandlung einer allergischen und/oder Autoimmun-Erkrankung nützlich ist, umfassend die folgenden Schritte:
- Bereitstellen einer ersten Gruppe von Zellen eines Typs, der den CD36-Rezeptor exprimiert,
- Exponieren der Zellen gegenüber einem Cystatin, das von einer Nematode abgeleitet ist, wobei das Cystatin und die Nematode wie in einem der Ansprüche 1-21 definiert sind,
- Nachweisen und quantitatives Bestimmen, als ein erstes Signal, des Ausmaßes der Bindung zwischen dem Cystatin und dem CD36-Rezeptor,
- Bereitstellen einer zweiten Gruppe von Zellen, des selben Typs wie die erste Gruppe von Zellen,
- Exponieren der zweiten Gruppe von Zellen gegenüber einer Kandidatenverbindung,
- Nachweisen und quantitatives Bestimmen, als ein zweites Signal, des Ausmaßes der Verbindung zwischen der Kandidatenverbindung und dem CD36-Rezeptor,
- Vergleichen des ersten Signals mit dem zweiten Signal und
- Identifizieren der Kandidatenverbindung als einer Kandidatenarznei zur Verhinderung und/oder Behandlung einer allergischen und/oder Autoimmun-Erkrankung, wenn das Ausmaß der Bindung, das mittels des zweiten Signals quantitativ bestimmt worden ist, gleich oder größer als das Ausmaß der Bindung ist, welches durch das erste Signal quantitativ bestimmt worden ist.

## Revendications

1. Cystatinedérivéed'unnématode parasite destinée à être utilisée dans un procédé pour la prévention et/ou le traitement d'une maladie allergique et/ou auto-immunechez un patient.

2. Cystatinedestinée à êtreutiliséeselon la revendication 1, **caractérisée en ce que** leditnématode parasite est parasite pour les humains.

3. Cystatine destinée à êtreutiliséeselon la revendication 1, **caractérisée en ce que** leditnématode parasite est parasite pour les animaux.

4. Cystatinedestinée à êtreutiliséeselon la revendication 3, **caractérisée en ce que** leditnématode parasite est parasite pour les animaux canins, de préférence les chiens, ouest parasite pour les rongeurs, de préférence les souris, ouest parasite pour les animaux félins, de préférence les chats.

5. Cystatinedestinée à être utilisée selon l'une quelconque des revendications 1-4, **caractérisée en ce que** leditnématodeestsélectionnédans le groupe comprenant Onchocercavolvulus, Brugiamalayi, Wuchereriabancrofti, Loa loa et Acanthocheilonemaviteae, Dirofilariaimmitis, Dirofilariarepens, Nippostrongylusbrasiliensis et Litomosoidessigmodontis.

6. Cystatinedestinée à être utilisée selon l'une quelconque des revendicationsprécédentes, **caractérisée en ce que** laditemaladieallergique et/ou auto-immuneestsélectionnéedans le groupecomprenant des maladiesallergiques des organes respiratoires comme l'asthme, le rhume des foins, la sinusite allergique, la rhiniteallergique, du systèmegastro-intestinal comme des allergiesalimentaires, de la peaucomme la dermatiteatopique, des maladiesallergiquessystémiquescomme des réactionsanaphylactiques, des maladies auto-immunes des articulations et/ou de la peau et/ou des organes internes comme la polyarthriterhumatoïde, la psoriasis, le lupus érythémateux, la sclérose en plaques, et des maladies intestinales inflammatoirescomme la coliteulcéreuse et la maladie de Crohn.

7. Cystatinedestinée à êtreutiliséeselon la revendication 6, **caractérisée en ce que** laditemaladieallergiqueestl'asthmeou le rhume des foins.

8. Cystatinedestinée à êtreutiliséeselon la revendication 6, **caractérisée en ce que** laditemaladieintestinaleinflammatoireest la colite, de préférence la coliteulcéreuse.

9. Cystatinedestinée à être utilisée selon l'une quelconque des revendicationsprécédentes, **caractérisée en ce que** laditecystatinepossède une séquence sélectionnée dans le groupecomprenant SEQ ID NO: 1 (cystatine de Acanthocheilonema vitae L43053), SEQ ID NO: 2 (cystatine de Onchocercavolvulus M37105), SEQ ID NO: 3 (cystatine de Brugiamalayi) et des séquencesquisont 70 % identiques, de préférence 80% identiques, de manière davantage préférée 90 % identiques et, de la manière la plus préférée entre toutes, 99 % identiques à l'unequelconque des susditesséquences.

10. Cystatinedestinée à être utilisée selon l'une quelconque des revendications précédentes, **caractérisée en ce que** laditecystatineest une cystatine recombinante et a étéproduite par un systèmed'expressionprocaryoteoueucaryote.

11. Cystatine destinée à être utilisée selon l'une quelconque des revendicationsprécédentes, **caractérisée en ce que** laditemaladieestassociée à une numération des cellulessanguineséosinophilesaugmentée et/ou à un taux d'IgE augmenté, par comparaison à un patientquineprésentepasladitemaladie, et leditmédicament, suite à son administrationauditpatient, entraîne une réduction de laditenumération des cellulessanguineséosinophilesaugmentée et/ou une réduction dudit taux d'IgE augmenté, de préférence à une numération des cellulessanguineséosinophiles et/ou à un taux d'IgE d'un individu en bonnesanté.

12. Cystatine destinée à êtreutiliséeselon la revendication 11, **caractérisée en ce que** laditenumération des cellules sanguines éosinophiles augmentée est> 4 % de tous les globulesblancssanguinsd'unpatientou un nombre total de cellules sanguines éosinophiles> 360/µl dans le sang périphérique d'un patient, et ledit taux d'IgE augmenté est un tauxsérique> 100 kU/l chez un patientadulte.

13. Cystatine destinée à être utilisée selon l'une quelconque des revendicationsprécédentes, **caractérisée en ce que** laditecystatineestadministréeauditpatient en tant que protéine.

14. Cystatinedestinée à être utilisée selon l'une quelconque des revendications 1-12, **caractérisée en ce que** laditecystatineestadministréeauditpatient en tant qu'acide nucléique codant pour laditecystatine.

15. Cystatinedestinée à être utilisée selon l'une quelconque des revendicationsprécédentes, **caractérisée en ce que** laditecystatineestadministrée par voiesystémiqueauditpatient, de préférence par injection, inhalation et/ou une autreincorporationcomme une ingestion.

16. Cystatinedestinée à être utilisée selon l'une quelconque des revendications précédentes, **caractérisée en ce que** laditecystatineestadministréeauditpatient par voieintranasale, intrapulmonaire, intrapéritonéale, intrathécale, intra-lésionnelle, sous-cutanée et/ouintramusculaire.

17. Cystatinedestinée à être utilisée selon l'une quelconque des revendications précédentes, **caractérisée en ce que** laditecystatineestadministrée en combinaisonavec un autremédicamentsélectionnédans le groupeconsistant en des médicaments anti-inflammatoirescomme des corticostéroïdes, des médicaments anti-inflammatoires non stéroïdiens, et/ou des antihistaminiques.

18. Cystatinedestinée à être utilisée selon l'une quelconque des revendications précédentes, **caractérisée en ce que** leditpatientest un mammifère, de préférence un être humain.

19. Cystatinedestinée à être utilisée selon l'une quelconque des revendications 1-17, **caractérisée en ce que** leditpatientest un animal.

20. Cystatinedestinée à êtreutiliséeselon la revendication 19, **caractérisée en ce que** ledit animal est un animal canin, de préférence un chien, ouest un animal félin, de préférence un chat, ouest un rongeur, de préférence une souris.

21. Cystatinedestinée à être utilisée selon l'une quelconque des revendicationsprécédentes, **caractérisée en ce que** laditecystatineestutilisée pour se lieraurécepteur CD36.

22. Procédé de criblaged'unmédicamentcandidatutile pour la prévention et/ou le traitement d'une maladie allergique et/ou auto-immune, comprenant les étapessuivantesconsistant à :
- mettre à disposition un premier groupe de cellules d'un type exprimant le récepteur CD36,
- exposerlesditescellules à une cystatine dérivée d'un nématode, laditecystatine et leditnématodeétantdéfinistels que dansl'unequelconque des revendications 1-21,
- détecter et quantifier, en tant que premier signal, l'étendue de la liaison entre laditecystatine et leditrécepteur CD36,
- mettre à disposition un secondgroupe de cellules du même type que le premier groupe de cellules,
- exposerleditsecondgroupe de cellules à un composécandidat,
- détecter et quantifier, en tant que secondsignal, l'étendue de la liaison entre leditcomposécandidat et leditrécepteur CD36,
- comparerledit premier signal avec ledit second signal, et
- identifierleditcomposécandidat en tant que médicamentcandidat pour la prévention et/ou le traitement d'une maladie allergique et/ou auto-immune si l'étendue de la liaison quantifiée par leditsecondsignalestsupérieureouégale à l'étendue de la liaison quantifiée par ledit premier signal.
